# EUROPEAN PATENT SPECIFICATION

(11) **EP 1 855 754 B1**
(45) Date of publication and mention of the grant of the patent: **02.09.2009**
(21) Application number: 06709700.6
(22) Date of filing: 09.02.2006
(51) Int. Cl.: A61M 25/02

(54) **A device for fixating a tube relative to a skin surface and assembly**
Vorrichtung zur fixierung eines Schlauchs relativ zu einer Hautoberfläche und Anordnung
Dispositif de fixation d'un tube relativement à peau et assemblage

(30) Priority: 11.02.2005 EP 05250777; 15.02.2005 US 653193 P
(43) Date of publication of application: 21.11.2007
(73) Proprietor: Unomedical Limited, Redditch Worcestershire B98 9DW (GB)
(72) Inventor: SOUTHWELL, James Edward, Brockworth, Gloucester GL3 4RH (GB)
(74) Representative: Hoffmann Dragsted
(86) International application number: PCT/GB2006/000462
(87) International publication number: WO 2006/085085

(56) References cited:
- EP-A- 0 424 275
- WO-A-97/21459
- WO-A-99/24103
- WO-A-99/59671
- WO-A-02/065976
- GB-A- 2 128 481
- US-A- 4 981 475
- US-B1- 6 231 547

## Description

The present invention generally relates to the technique of applying and fixating a tube, such as a drainage tube, relative to a skin surface part of a patient or person, and more precisely a device for fixating such tube relative to a skin surface part of a patient or person, and an assembly comprising the device and the tube.

It is a well-known technique to enter a tube through the skin of a patient or person in order to drain liquid from a cavity within the patient or person, e.g. after a surgical operation. In certain applications, the tube is also used for creating a vacuum within the cavity which is drained by means of the tube. An example of such an application is the treatment of a collapsed lung, in which application the cavity of the thorax of the patient in question is evacuated through a pleural tube in order to cause an adhesion of the pulmonary pleura of the patient to the costal pleura of the patient. In all applications of tubes, such as drainage tubes, there exists a risk that the tube is blocked as the tube is bent, or kinks of the tube are produced. An alternative application of a tube not being a drainage tube in the administration of a drug or other medical fluid.

Provided the tube is to be vented for creating a vacuum within the cavity which is vented through the tube, a further problem exists in creating a reliable and lasting sealing of the entrance of the tube through the skin of the patient, and further an overall desire of rendering it possible to create a sealing and a fixation of the tube in a swift and reliable manner. Hitherto, the tube, such as a pleural tube which is used for venting the cavity of the thorax of the patient, the lung of whom has collapsed, has been fixated and sealed in a manual operation in which the medical doctor who applies the pleural tube arranges one or more vaseline-impregnated wads round the pleural tube at the entrance of the pleural tube through the skin of the patient and compresses the vaseline-impregnated wads in order to create a sealing at the entrance. Thereupon, the vaseline-impregnated wads and the pleural tube as well are fixated by means of plaster.

The technique of applying and fixating a tube, in particular a venting drainage tube, such as a pleural tube, is a complex and time-consuming operation which requires skill and which further often turns out to be inadequate and inappropriate as the sealing of the entrance of the pleural tube through the skin of the patient in question leaks, causing great harm to the patient.

Various devices for fixating a tube have recently been developed as disclosed in eg. US patent no. 4,419,094, US patent no. 4,645,492, WO 91/07204. WO 93/17738, WO 93/25264, WO 95/33508 and WO 97/21459.

When using these known devices, a proximal part of the tube extending from the body of the patient through a through-going passage in the device, is bent to form a loop whereby a distal part of the tube is fixated to the skin surface of the patient by means of eg. a separate piece of plaster. This procedure, however, gives rise to a potential risk of the tube becoming blocked if the tube is inadvertently doubled or bent

upon itself. US Patent no. 6,231,547 is also known in the art.

In WO 95/33508 and WO 97/21459, a device for fixating a tube is known, which device to a great extent eliminates the drawbacks of the earlier known devices. The device known from these two published international patent applications on the basis of which numerous patents have issued, now assigned to the applicant company, may, however, from a technical view as to the ability to fixate and maintain the tube in a preset position be refined, further taking into consideration the necessity of allowing the device fixating and maintaining the tube to be exposed to mechanical pressure or forces, as the patient or person wearing the tube fixated by means of the device may e.g. shift his or her position lying in a bed and in doing so, lie on the device which should then be capable of accumulating the pressure or force generated by the person or patient shifting a lying position and at the same time, maintain the tube safely and securely in its intentional position.

The devices known from the above WO publications, WO 95/33508 and WO 97/21459, may further be refined, in particular in view of the necessity of sterilizing the device, as the commonly used foamed materials or PU based materials may not stand exposure to sterilizing gasses conventionally used within the surgical dressings industry, such as gasses, e.g. T-gas, also know as ethylene oxide gas, formaldehyde, propylene oxide and other chemical sterilizing gasses.

An object of the present invention is to provide a device for fixating a tube, which device may be configurated so as to have specific properties dedicated to a specific area and different properties, as to compressibility and flexibility dedicated to a different area of the device.

A further object of the present invention is to provide a device for fixating a tube, which device allows the entire device after manufacture to be sterilized by means of a sterilizing gas, such as T-gas or ethylene oxide gas without deteriorating or in the alternative ruining the device or the performance of the device, as the device is used for its intentional purpose of fixating and maintaining a tube in a specific and fixed position relative to the skin surface of a patient or person.

A still further object of the present invention is to provide a device for fixating a tube, which device to any substantial extent eliminates the risk that the tube is blocked at the entrance of the skin of the patient in question by to any substantial extent eliminating the risk of creation of kinks of the tube.

A still further further object of the present invention is to provide a device for fixating a tube which device constitutes a self-adhering devices for adhering to the skin of the patient in question still providing a structure allowing the device to be handled and positioned properly relative to the tube and also the skin of the patient in question without any substantial risk of adhering the device unintentionally to the tube or the skin of the patient in question.

An advantage of the device according to the present invention relates to the supporting component of the device according to the invention, which component is a unitary structure exhibiting specific mechanical properties as to compressibility and flexibility dedicated to a specific supporting area and different properties dedicated to a different supporting area of the device.

A further advantage of the device according to the present invention lies in that the device is a self-adhering device which is applied to the tube and to the skin of the patient in question without any additional fixation means such as supplementary plasters, clamps etc., and is easily positioned in the intentional position and orientation relative to the tube and the skin of the patient In question before fixating the device relative to the skin of the patient in question.

A feature of the device according to the present invention lies in that the device according to the present invention constitutes a disposable unitary structure which has been presterilized by means of a sterilizing gas, such as T-gas or ethylene oxide gas, and is applied as a plaster structure to the skin of the patient.

The above object, the above advantage, and the above feature together with numerous other objects, advantages, and features which will be evident from the below detailed description of preferred embodiments of the device according to the present invention are obtained by means of a a device for fixating a tube, such as a drainage tube, relative to a skin surface part of a patient or person, comprising in accordance with the present invention as defined is independent claim 1.

The support component of the device according to the present invention fulfils the main purposes of turning the length of the tube which length extends outwardly from the aperture of the plaster component and outwardly from the skin surface part of the person or patient to which skin surface part the device is fixated by means of the plaster component from an orientation substantially perpendicular to the skin surface part of the patient or person to an orientation substantially parallel with and in contact with the skin surface part of the patient or person and also of preventing the tube from being blocked by the turning from the above perpendicular orientation to the above parallel orientation or through the generation of kinks or bends on the tube. A basic realization of the present invention relates to the fact that the support component is made from a thin shell of a plastics material which, due to its compressibility and flexibility, allows the support component to be deformed or compressed at specific areas, whereas different areas, such as a part of the outer surface part supporting the tube may not be compressed or deformed, as the above-mentioned different areas are reinforced by one or more reinforcing elements.

The reinforcing element characteristic of the device according to the present invention may be implemented in accordance with different techniques, as the reinforcing element may be constituted by an integral component of the shell constituting the support component, or alternatively, be a separate component to be positioned within the support component or a reinforcing element positioned within the shell or received within the plastics materials of the shell, such as a reinforcing wire, thread or mesh.

According to the presently preferred embodiments of the device according to the present invention, the reinforcing element is an integral reinforcing rib positioned within the shell and extending along and possibly parallel with at least a part of the surface part or alternatively, the reinforcing element may be constituted by an integral reinforcing rib positioned within the shell and below at least a part of the outer surface part.

According to a particular feature of the device according to the present invention, the device is adapted to the ability of standing exposure to a sterilizing gas, such as T-gas or ethylene oxide gas, commonly used within the wound dressing industry and the shell constituting the support component of the device according to the present invention is preferably made from a plastics material capable of standing exposure to the sterilizing gas, such as T-gas.

The material used for the support component may be constituted by any appropriate plastics material fulfilling the basic requirements as to exhibiting flexibility and elasticity for allowing the shell to be collapsed or deformed and, possibly and advantageously, also the capability of the material to be able to stand exposure to a sterilizing gas. An example of such material is a copolymer of ethylene butyl acrylate, e.g. the copolymer supplied from the company DuPont™ under the name Elvaloy® 3427AC.

For obtaining the desired ability as to allow the shell to be collapsed and deformed, the shell may have a thickness of approximately 0.5 - 1.5 mm, such as 0.6 - 1.0 mm, e.g. approximately 0.6 - 0.8 mm, or a thickness of 0.6 - 0.7 mm, 0.7 - 0.8 mm, 0.8 - 0.9 mm, 0.9 -1.0mm, 1.0-1.1 mm, 1.1 - 1.2 mm, 1.2-1.3mm, 1.3-1.4mm or 1.4-1.5mm.

The support component may constitute a dome shaped structure, which may be fixated to the plaster component through a connecting component such as a thin adhesive foil. Alternatively and preferably, the support component is provided with an integral flange extending outwardly from the central dome shaped part of the support component and constituting a connecting component for connecting the central dome shaped part of the support component to the plaster component.

In the above described embodiment having a connecting component or alternatively and preferably an integral flange of the support component constituting the connecting component an aperture is provided in the connecting component or the integral flange, which aperture is positioned or located in registration with the aperture of the plaster component.

In order to obtain the desired compressibility of the shell of the support component characteristic of the present invention, a through-going hole is preferably provided in the shell constituting the support component, which hole allows gas, such as atmospheric air to escape when the shell is compressed, thereby preventing the air from being trapped within the inner space defined within the shell and in doing so, establishing a deformable balloon.

The inner reinforcing element or reinforcing rib of the shell constituting the supporting component characteristic of the present invention may extend along the entire length of the shell or the outer surface part thereby preventing the support component from being compressed or deformed at the area around the reinforcing element or reinforcing rib. in the presently preferred embodiment of the device according to the present invention, a part of the outer surface part, namely the part adjacent to or closest to the aperture of the plaster component is unsupported by the reinforcing element or the inner supporting rib for allowing the part of the shell constituting the support component adjacent to the aperture of the plaster component to be slightly compressed or deformed and in doing so, allow the device to accommodate to e.g. the pressure generated, as a patient or person lies on the device.

Provided a specie area of the shell is unsupported or not reinforced by a reinforcing element or an inner reinforcing rib, one or more distance elements may be provided at the area in question, which distance elements are positioned within the shell and have a reduced height as compared to the free height within the shell at the area in question, thereby delimiting the possible height of compression of the shell at the area in question.
These distance elements may, like the reinforcing element characteristic of the present invention be constituted by separate or loose distance elements or be integrally produced with the shell.

The provision of a reinforcing element or reinforcing rib within the shell according to the teachings of the present invention may involve the provision of a single reinforcing rib or alternatively and advantageously, the provision of two or more reinforcing elements or reinforcing ribs, since the provisions of two or more reinforcing elements or reinforcing ribs allows more than a single area to be reinforced for obtaining a specific strength of the area or areas in question.

The outer surface part in which the tube is supported may have any appropriate configuration such as a curved configuration as viewed in the longitudinal direction of the tube, however, by the provision of the reinforcing rib positioned below the outer surface part supporting the tube, the outer surface may have a simple rectilinear configuration along the longitudinal direction of the tube rather than being bent or configurated into a zig zag. For properly fixating and maintaining the tube relative to the support component, the outer surface part of the support component may be configurated so as to provide lateral support of the tube, or alternatively, the device may be provided with securing means for securing the length of the tube relative to the support component. The securing means may be configurated as an integral part of the support component such as an adhesive coating or alternatively be provided by an integral flap of the plaster component, which flap is to be adhered to the top surface of the support component and optionally to the exposed outer surface of the length of the tube. Alternatively, a separate adhesive strip may be used for fixating the tube relative to the skin surface of the patient, relative to the plaster component and/or relative to the support component.

Provided a separate adhesive strip or a part of the plaster component be used for fixating the tube relative to the support component, the adhesive strip or the part of the plaster component in question may advantageously be provided with a transparent area at least in the area of the aperture of the plaster component for allowing visible inspection of the fixation of the length of the tube.

The above object, the above advantage and the above feature together with numerous other objects, advantages and features, which will be evident from the below detailed description of the preferred embodiments of the device according to the present invention are obtained by means of an assembly of a tube and the device according to the present invention and having any of the above described features of the device. The drainage tube may be any tubular elements such as a rubber or plastics material tube or a so-called 'pig-tall' type drainage tube.

The present invention will now be further described with reference to the drawings, in which
Fig. 1 is a schematical view of a first and presently preferred embodiment of a tube fixating device according to the present invention,
Figs. 2 and 3 are schematical views illustrating the technique of applying the first embodiment of the device shown in Fig. 1,
Fig. 4 is a top view of a first embodiment of a two-part component of the tube fixation device according to the present invention shown in Figs. 1-3,
Fig. 5 is a sectional view of the component shown in Fig. 4 of the device according to the present invention shown in Figs. 1-3,
Fig. 6 is a bottom view of the component shown in Fig. 4 of the device according to the invention,
Fig. 7 is a sectional view similar to the view of Fig. 5 of the component shown in Figs. 4 - 6 of the device according to the present invention,
Fig. 8 is a sectional view similar to the view of Fig. 5 of a second embodiment of the two-part component of the tube fixation device shown in Figs. 1-3,
Fig. 9 is a bottom view similar to the view of Fig. 6 of the second embodiment of the two-part component shown in Fig. 8,
Fig. 10 is a sectional view similar to the view of Fig. 7 of the second embodiment of the two-part component shown in Fig. 8,
Fig. 11 is a sectional view similar to the views of Figs. 6 and 8 of a third and presently preferred embodiment of the two-part component of the tube fixation device shown in Figs. 1-3,
Fig. 12 is a bottom view similar to the views of Figs. 6 and 9 of the third and presently preferred embodiment of the two-part component shown in Fig, 11, and
Fig. 13 is a sectional view simliar to the views of Figs. 7 and 10 of the third and presently preferred embodiment of the two-part component shown in Fig. 8.

The first embodiment of the device for fixating a tube, such as a drainage tube according to the present invention serves the overall purpose of ensuring that the tube does not become blocked due to the tube inadvertently being doubled or bent upon itself, thereby providing a tube fixating device which render it extremely simple and far less complicated than bitherto to fixate the tube to a patient, further having specific compressible areas and different non-compressible areas, and further serves the of allowing the device to be easily stérilized by exposure to a sterilizing gas such as T-gas or ethylene oxide gas conventionally used in the surgical dressings industry.

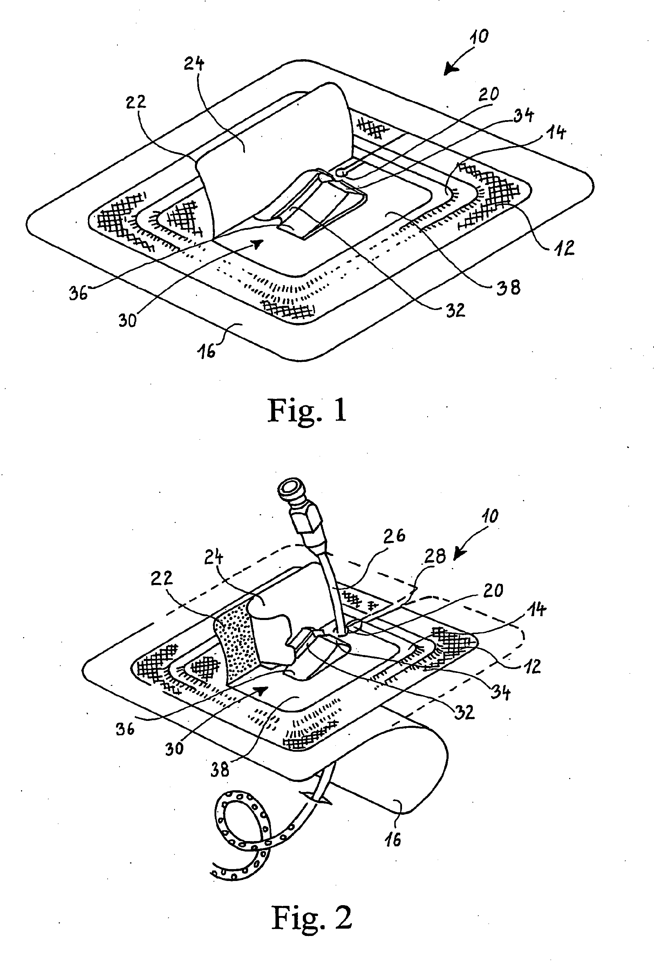

As shown in Fig. 1 and as will be described in further details below, the device according to the first embodiment of the invention designated the reference numeral 10 in its entirety generally comprises plaster component 12 having a central aperture 14 and carrying an adhesive layer on the lower side thereof by means of which the plaster component 12 adheres to the skin surface of the patient to whom the device 10 is applied.

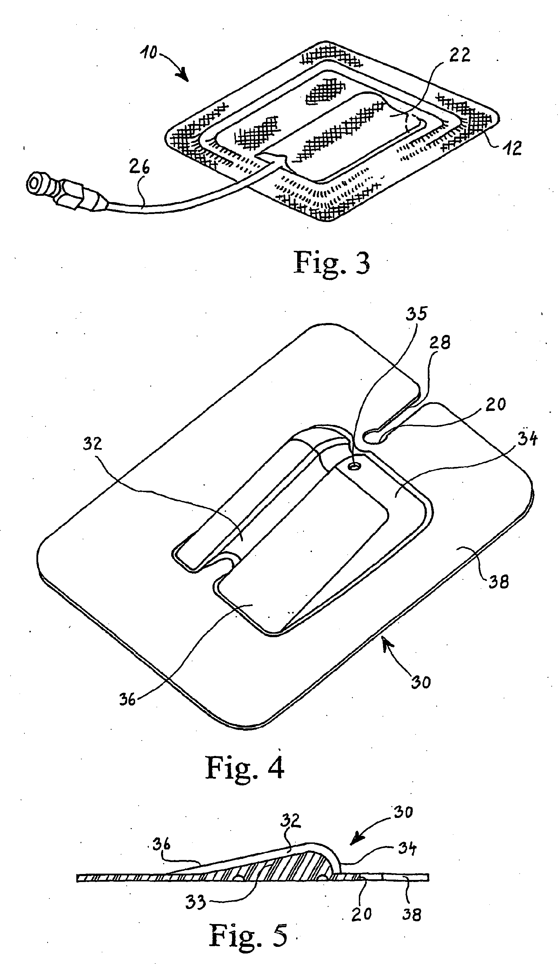

The device 10 further comprises a cover sheet 16 covering said adhesive layer, and an integrally cast support component 30 to be described in greater details below with reference to Figs. 4 - 6 and having a central done and an outwardly protruding flange or connecting part 38. The flange or connecting part 38 has an aperture 20 and the donne has a groove 32 for receiving a tube of the support component 30 protrudes from the upper surface of the plaster component 12 through a central aperture 14 of the plaster component.

As shown, the dome of the support component 30 is generally termed with a rounded or curved upper surface composed, of two sections, a first section 34 constituting a front surface section positioned adjacent to the aperture 20 and a second section 36 positioned or located behind the first section 34. The groove 32 extends continuously from the first. section 34 to and through the second section 36 and provides by the overall configuration of the first and second sections 34 and 36, respectively, of the support component 30, a support surface for the tube as the groove part of the first section 34 supports the tube ss the tube is turned from an orientation substantially perpendicular to the skin surface the patient to an orientation substantially parallel with the skin surface, whereas the groove part of the second section 36 provides a continuous support of the tube, as the tube is lowered into contact with the skin surface of the patient. The plaster component 12 has, as is illustrated in Fig. 1, a flap 22 provided as a cutaway part of the plaster component providing the aperture 14 and is at ist lower surface provided with a further cover sheet 24 covering the adhesive of the plaster component flap 22,

As shown in Fig. 2, illustrating the fixation of the device 10 according to the present invention to body of the patent, the outer end of a tube 26 which is a so-called pig-tail type drainage tube is inserted through the aperture 20 of the support component 30, i.e. a tube which is provided with a distal end which is formed into the configuration of a pig-tail and which to a high degree resists the autonomous expelling of foreign bodies from the human body as compared to a straight line drainage tube.

Alternatively, the two-parts of the device 10 delimited by the slit 23 extending from the aperture 20 to the periphery of the device may be forced apart thus allowing the tube to be entered into the aperture 20, During the process of applying the device 10 to the tube 26 and fixating the device 10 relative to a skin surface part of a of parent circumferentially encircling the tube 26, the device 10 is initially threaded on the tube or alternatively shapped round the tube 26 utilizing the slit 28, whereupon the entire device 10 is property positioned relative to the skin surface part, and the cover sheet 16 is then removed for exposing the adhesive layer to be adhered to said skin surface part as the adhesive layer is brought into facial contact with the outer skin surface the patient.

Fig. 3 shows the device 1C according to the invention, after the plaster component has been fixated to the body of the patient in this next step, the tube 26 is turned to a position in which it extending substantially parallel to the skin surface of the patient and in contact with the skin surface, in this the tube 24 is supported by the support component 30, The tube 26 is taken up by the groove 32 and the cover sheet 24 of a flap 22 is removed. The flap 22 is then moved to the position shown on Fig. 3 in which the lower side of the flap 22 is in contact with the upper surface of the support component 30 and the tuba 26, causing the tube 26 to be secured and maintained in place. In Figs. 4-7, the integrally cast support component 30 is shown in greater details, which component is made from a thin shell of deformable plastics material capable of standing exposure to ethylene oxide for an extended period of time, thereby allowing that the entire device may be sterilized after the device has been assemble.

As is evident from Fig. 4, the front section 34 of the support component 30 of the Integrally cast structure is provided with a venting aperture 35 allowing the support component 30 to be slightly compressed allowing any gas, such as atmospheric air contained within the support component 30 to escape through the venting aperture 35. According to a particular feature of the integrally cast two-part structure shown in Fig. 4, a reinforcing rib is provided at the bottom surface of the groove 32, which rib is shown in Fig. 5 and designated the reference numeral 33. The rib 33 extends to a position close to the front section 34. for reinforcing and supporting the groove 32 in its entirety, as shown in Fig. 5, serving to support the entire groove 32 for preventing that the groove of the support component 30 be collapsed by e.g. a patient intentionally or unintentionally applying a pressure to the device 10 according to the present invention.

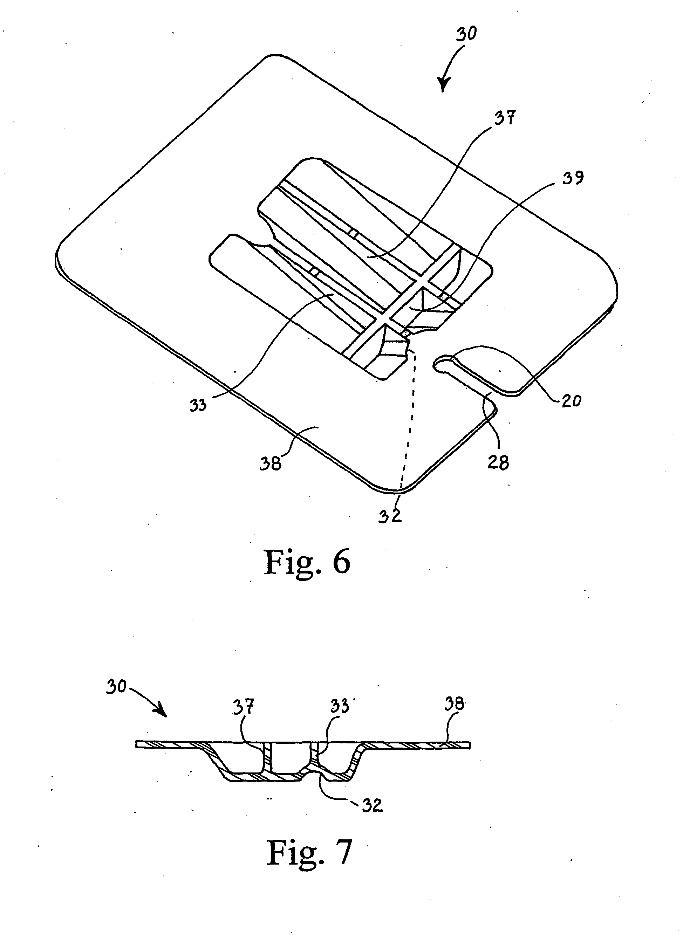

Apart from the one reinforcing rib 33 positioned below the groove 32, additional reinforcing ribs may be provided, as is illustrated in Figs. 6 and 7, in which the support component 30 is shown from the bottom surface prior to applying the integrally cast structure assembly to the plaster component 12 and disclosing the one reinforcing rib 33 positioned below the groove 32 and further disclosing an additional reinforcing rib 37 extending parallely with the reinforcing rib 33 It is to be understood that the reinforcing rib 37 may have a configuration different from the configuration of the rib 33. In Fig. 7, a vertical sectional view of the integrally cast structure of the support component 30 is shown.

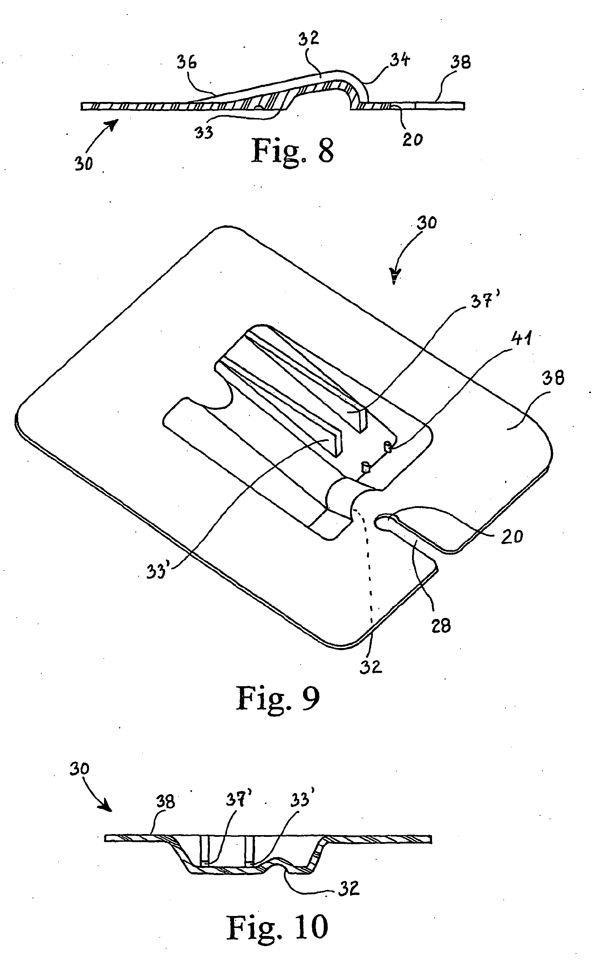

In Figs: 8-10, a second embodiment of the support component 30 is shown, which differs from the above-described first embodiment shown In Figs. 8-4-7 in that the reinforcing rib 33 is shifted from a position below the groove 32, to a position adjacent to and parallel with the groove. Furthermore, the ribs 33' and 37' shown in Figs. 8-10 have a reduced length as compared to the ribs 33 and 37 shown in Figs. 5-7. In the embodiment shown in Figs. 8-10, a set of distance elements or bumps 41 is provided, which distance elements serve to limit the maximum height of compression of the area in which the distance elements are provided and consequently, serve to prevent a complete compression or depression of the area in which the distance elements or bumps are provided.

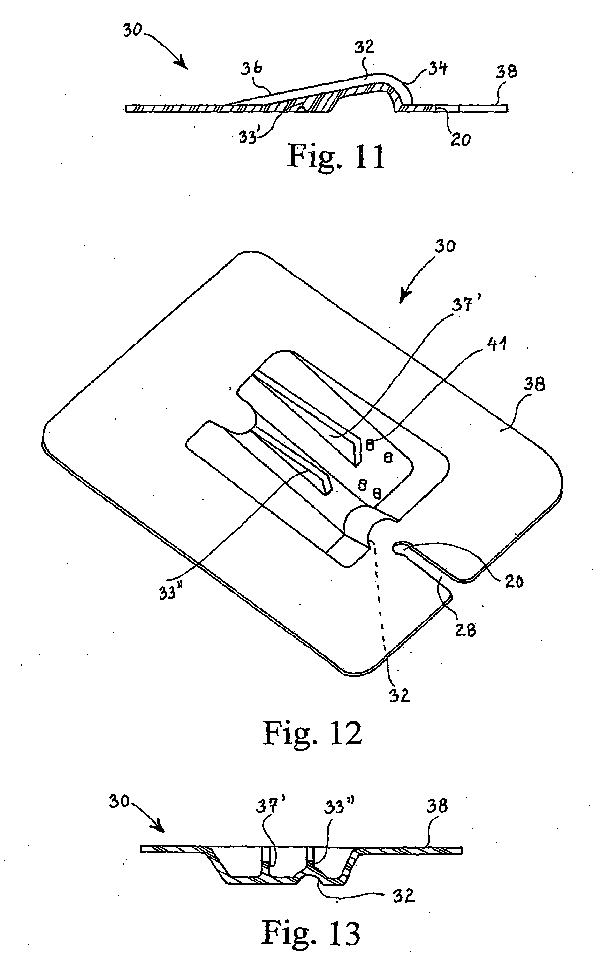

In Figs. 11-13, a third and presently preferred embodiment of the support component 30 is shown, which third embodiment is a combination of the above-described first and second embodiments in that, in the third and presently preferred embodiment, the rib 33' is shifted as compared to the embodiment shown in Fig. 9 from a position adjacent to the groove 32 to a position below the groove 32 similar to the position of the groove 32 of the first embodiment shown in Fig. 6. In addition, a total of four distance elements or bumps 41 is provided,

### Example

A prototype implementation of the presently preferred embodiment of the device according to the present invention shown in Figs. 1 -13 and 8-10 designated the reference numeral 10, was made from the following components.

The support component 30 was integrally cast from a copolymer of ethylene and butyl acrylate of the type Elvaloy® 3427AC suppled from DuPont^{™} providing an integral shell structure, Its dimensions were 60 × 50 mm and the integrally cast shell structure had a thickness of 0.6 mm. The central dome of the support component 30 measured 25 × 31,5 mm and the maximum height of the dome was 5.9 mm. The inner diameter of the aperture 20 was 4 mm. The slit 28 had a length of 5 mm. The plaster component 12 was formed from non-woven polyamide, and the central opening or aperture 14 measured 30 × 50 mm. The layer of a hydro-colloidal material, having a thickness of 1 mm, was applied to the lower surface of the plaster component 12. The cover sheets 16 and 24 were constituted by siliconized paper.

It is to be understood that the above-described embodiments of the device for fixating a tube, such as a drainage tube, the assembly and also the two-part component characteristic of the device according to the present invention may be modified in numerous ways. In particular, the two-part component described above in three embodiments with reference to Figs. 4-13 may be modified, e.g. by combining the elements of one of the embodiments into a different embodiment and further by shifting the reinforcing ribs from e.g. the orthogonal orientation relative to the groove 32. Furthermore, the reinforcing ribs and the bumps may be proved by a separate element or separate elements to be combined with the shell constituting the two-part component or be incorporated into the material of the shell by e.g. molding or casting a reinforcing, wire, thread or mesh into the material of the shell. All such variances which will be evident to a person having ordinary skill in the art having read the present specification, are together with other modifications, obvious to the person having ordinary skill in the art, is to be construed part of the present invention as defined in the apending claims.

## Claims

1. A device (10) for fixating a tube (26) relative to a skin surface part of a patient or person; comprising:
a plaster component (12) including a support foil having an aperture (20) for receiving said tube (26), and opposite first and second side surfaces, said aperture (20) transversing said first and second side surfaces, said first side surface being provided with an adhesive layer for fixating said plaster component (12) relative to said skin surface part of said patient or person,
a support component (30) arranged at said second side surface of said plaster component (12) and protruding from said second side surface, said support component (30) being arranged adjacent to said aperture (20),
said support component (30) having an outer surface part for supporting a length of said tube (26) extending from said aperture (20) at said second side surface of said plaster component (12) and being configured so that said length of said tube (26) is turned from an orientation substantially perpendicular to said skin surface part of said patient or person to an orientation substantially parallel with said skin surface part of said patient or person,
the support component (30) is constituted by a thin shell of a plastics material which is elastically collapsible and deformable, and
said support component (30) and said plaster component (12) being joined together, the device (10) **characterized in that**
the outer surface part of the support component (30) is configurated so that said length of said tube (26), in supported relationship with said support component (30) and laterally supported by said outer surface part, is lowered into contact with the skin surface part of the patient and brought into facial contact with said plaster component (12) and said skin surface part,
the support component (30) defines an inner surface within said shell, and
the support component (30) comprises at least one inner reinforcing element (33, 33', 33", 37, 37'. 39), preventing the support component (30) from being compressed or deformed around said at least one reinforcing element (33, 33', 33", 37, 37', 39).

2. The device according to claim 1, said at least one reinforcing element (33, 33'. 33", 37, 37', 39) being a separate supporting or reinforcing element positioned within said shell or received within said plastics material of said shell.

3. The device, according to claim 1, said reinforcing element (33, 33', 33", 37, 37'. 39) being an Integral reinforcing rib positioned within said shell extending along and possibly parallel with at least a part of the outer surface part.

4. The device according to claim 1, said reinforcing element (33, 33', 33", 37, 37', 39) being positioned within said shell and below at least a part of said outer surface part.

5. The device according to any of the claims 1- 4, said plastics material being capable of standing exposure to a sterilizing gas, such as T-gas.

6. The device according to claims 4 or 5, said support component (30) having two or morse reinforcing elements or reinforcing ribs (33, 33', 33", 37, 37', 39).

7. The device according to any of the claims 1 - 6, said support component (30) having a through-going hole (35) through said shell for allowing gas to escape through said hole when elastically collapsing or compressing said shell.

8. The device according to any of the claims 1 - 7, said inner reinforcing element (33, 37) extending along the entire length of said outer surface part.

9. The device according to any of the claims 1 - 7, said outer surface part being unsupported by said inner reinforcing element adjacent to said aperture (20) of said plaster component (12).

10. The device according to any of the claims 1 - 9, said support component (30) further including one or more distance elements (41) positioned within said shell at one or more positions and delimiting the height of compression of said shell at said one or more positions.

11. The device according to any of the claims 1-10. said plastics material being a copolymer of ethylene butyl acrylate.

12. The device according to any of the claim 1 -11, said shell having a thickness of approximately 0.5 - 1.5 mm, such as 0.6 -1.0 mm, e.g. approximately 0.6 - 0.8 mm, or a thickness of 0.6 ~ 0.7 mm, 0.7 ~ 0.8 mm, 0.8 ~ 0.9 mm, 0.9 ~ 1.0 mm, 1.0 ~ 1.1 mm, 1.1 ~ 1.2 mm, 1.2 ~ 1.3 mm, 1.3~1.4 mm or 1.4 ~ 1.5 mm.

13. The device according to any of the claims 1 ~ 12 said plaster component end said support component (30) being joint together by means of a connecting component (38).

14. The device according to claim 13, said connecting component (38) constituting an integral flange of said support component (30).

15. The device according to any of the claims 13 or 14, said connecting component (38) being provided with an aperture, said aperture being in registration with said aperture (20) of said plaster component (12).

16. The device according to any of the claims 1 - 15, said outer surface part of said support component (30) being substantially rectilinear.

17. The device according to any of the claims 1 ~16, said support component (30) providing lateral support of said tube (26).

18. The device according to any of the claims 1 -17, further comprising securing means (22) for securing said length of said tube (26) relative to said support component (30).

19. The device according to claim 18, said securing means (22) forming an integral part of said support component (30).

20. The device according to claim 18, said securing means (22) being integrally connected to said plaster component (12).

21. The device according to any of the claims 16 ~ 20, said securing means (22) comprising an adhesive strip for fixating said tube (26).

22. The device according to any of the claims 18 - 21, said securing means (22) being provided with a transparent area in the area of the aperture (20) of said plaster component (12).

23. An assembly of a tube (26) and a device for fixating said drainage tube (26) relative to a skin surface part of a patient or person, said device having any of the features of the device according to any of the claims 1 - 22.

## Patentansprüche

1. Vorrichtung (10) zur Fixierung eines Schlauches (26) relativ zu einem Hautoberflächenteil eines Patienten oder einer Person, enthaltend:
einen Pflasterteil (12), enthaltend eine Trägerfolie, die eine Öffnung (20) für die Aufnahme des Rohres (26) sowie gegenüberliegende erste und zweite Seitenflächen aufweist, wobei die Öffnung (20) die ersten und zweiten Seitenflächen durchläuft und die erste Seitenfläche mit einer Haftschicht versehen ist, mit der der Pflasterteil (12) relativ zum Hautoberflächenteil des Patienten oder der Person fixiert wird,
einen Halteteil (30), der an der zweiten Seitenfläche des Pflasterteils (12) angebracht ist und von der zweiten Seitenfläche hervorragt, wobei der Halteteil (30) benachbart der Öffnung (20) angeordnet ist,
wobei der Halteteil (30) einen Außenoberflächenteil hat, der eine Länge des Schlauches (26) trägt, der sich von der Öffnung (20) an der zweiten Seitenfläche des Pflasterteils (12) erstreckt, und so beschaffen ist, dass die Länge des Schlauches (26) von einer Ausrichtung im wesentlichen senkrecht zum Hautoberflächenteil des Patienten oder der Person zu einer Ausrichtung im wesentlichen parallel zum Hautoberflächenteil des Patienten oder der Person gewendet ist,
der Halteteil (30) aus einer dünnen Schale eines Kunststoffmaterials besteht, das elastisch faltbar und verformbar ist, und
der Halteteil (30) sowie der Pflasterteil (12) miteinander verbunden sind,
wobei die Vorrichtung (10) **dadurch gekennzeichnet ist, dass**
der Außenoberflächenteil des Halteteils (30) so beschaffen ist, dass die Länge des Schlauches (26) in gehaltener Beziehung mit dem Halteteil (30) und mit seitlicher Halterung durch den Außenoberflächenteil in einen Kontakt mit dem Hautoberflächenteil des Patienten abgesenkt und in Flächenkontakt mit dem Pflasterteil (12) und dem Hautoberflächenteil gebracht wird,
der Halteteil (30) eine Innenoberfläche mit der Schale bildet, und
der Halteteil (30) wenigstens ein inneres Verstärkungselement (33, 33', 33", 37, 37', 39) enthält, das verhindert, dass der Halteteil (30) um das wenigstens eine Verstärkungselement (33, 33', 33", 37, 37', 39) zusammengedrückt oder verformt wird.

2. Vorrichtung nach Anspruch 1, bei der das wenigstens eine Verstärkungselement (33, 33', 33", 37, 37', 39) ein separates Halte- oder Verstärkungselement ist, das innerhalb der Schale angeordnet oder im Kunststoffmaterial der Schale aufgenommen ist.

3. Vorrichtung nach Anspruch 1, bei der das Verstärkungselement (33, 33', 33", 37, 37', 39) eine integrale Verstärkungsrippe ist, die innerhalb der Schale angeordnet ist und sich entlang und möglicherweise parallel mit wenigstens einem Teil des Außenoberflächenteils erstreckt.

4. Vorrichtung nach Anspruch 1, bei der das Verstärkungselement (33, 33', 33", 37, 37', 39) innerhalb der Schale und unter wenigstens einem Teil des Außenoberflächenteils angeordnet ist.

5. Vorrichtung nach einem der Ansprüche 1 bis 4, bei der das Kunststoffmaterial in der Lage ist, dem Einfluss eines Sterilisationsgases, wie etwa T-Gas, zu widerstehen.

6. Vorrichtung nach Anspruch 4 oder 5, bei der der Halteteil (30) wenigstens zwei Verstärkungselemente oder Verstärkungsrippen (33, 33', 33", 37, 37', 39) enthält.

7. Vorrichtung nach einem der Ansprüche 1 bis 6, bei der der Halteteil (30) ein Durchgangsloch (35) durch die Schale aufweist, um es einem Gas zu gestatten, durch das Loch zu entweichen, wenn die Schale elastisch gefaltet oder zusammengedrückt ist.

8. Vorrichtung nach einem der Ansprüche 1 bis 7, bei der sich das innere Verstärkungselement (33, 37) entlang der gesamten Länge des Außenoberflächenteils erstreckt.

9. Vorrichtung nach einem der Ansprüche 1 bis 7, bei der der Außenoberflächenteil nicht von dem inneren Verstärkungselement benachbart der Öffnung (20) des Pflasterteils (12) gestützt ist.

10. Vorrichtung nach einem der Ansprüche 1 bis 9, bei der der Halteteil (30) weiterhin wenigstens ein Abstandselement (41) enthält, das innerhalb der Schale an wenigstens einer Stelle angeordnet ist und die Kompressionshöhe der Schale an wenigstens einer Stelle begrenzt.

11. Vorrichtung nach einem der Ansprüche 1 bis 10, bei der das Kunststoffmaterial ein Copolymer aus Äthylenbutylacrylat ist.

12. Vorrichtung nach einem der Ansprüche 1 bis 11, bei der die Schale eine Dicke von etwa 0,5 bis 1,5 mm, wie etwa 0,6 bis 1,0 mm, z.B. etwa 0,6 bis 0,8 mm oder ein Dicke vom 0,6 bis 0,7 mm, 0,7 bis 0,8 mm, 0,8 bis 0,9 mm, 0,9 bis 1,0 mm, 1,0 bis 1,1 mm, 1,1 bis 1,2 mm, 1,2 bis 1,3 mm, 1,3 bis 1,4 mm oder 1,4 bis 1,5 mm hat.

13. Vorrichtung nach einem der Ansprüche 1 bis 12, bei der der Pflasterteil (12) und der Halteteil (30) mit Hilfe eines Verbindungsteils (38) verbunden sind.

14. Vorrichtung nach Anspruch 13, bei der der Verbindungsteil (38) einen integralen Flansch des Halteteils (30) bildet.

15. Vorrichtung nach einem der Ansprüche 13 oder 14, bei der der Verbindungsteil (38) mit einer Öffnung versehen ist, wobei diese Öffnung mit der Öffnung (20) des Pflasterteils (12) ausgerichtet ist.

16. Vorrichtung nach einem der Ansprüche 1 bis 15, bei der der Außenoberflächenteil des Halteteils (30) im wesentlichen geradlinig ist.

17. Vorrichtung nach einem der Ansprüche 1 bis 16, bei der der Halteteil (30) einen seitlichen Halt für den Schlauch (26) bietet.

18. Vorrichtung nach einem der Ansprüche 1 bis 17, weiterhin enthaltend eine Befestigungseinrichtung (22), um die Länge des Schlauches (26) relativ zum Halteteil (30) zu befestigen.

19. Vorrichtung nach Anspruch 18, bei der die Befestigungseinrichtung (22) einen integralen Teil des Halteteils (30) bildet.

20. Vorrichtung nach Anspruch 18, bei der die Befestigungseinrichtung (22) integral mit dem Pflasterteil (12) verbunden ist.

21. Vorrichtung nach einem der Ansprüche 18 bis 20, bei der die Befestigungseinrichtung (22) einen Haftstreifen zum Fixieren des Schlauches (26) enthält.

22. Vorrichtung nach einem der Ansprüche 18 bis 21, bei der die Befestigungseinrichtung (22) mit einem transparenten Bereich im Bereich der Öffnung (20) des Pflasterteils (12) versehen ist.

23. Anordnung eines Schlauches (26) und einer Vorrichtung zum Fixieren des Drainageschlauches (29) relativ zu einem Hautoberflächenteil eines Patienten oder einer Person, wobei die Vorrichtung ein beliebiges der Merkmale der Vorrichtung nach einem der Ansprüche 1 bis 22 hat.

## Revendications

1. Dispositif (10) de fixation d'un tube (26) par rapport à une partie de surface cutanée d'un patient ou d'une personne comprenant :
un composant sparadrap (12) comportant une feuille support ayant une ouverture (20) destinée à recevoir ledit tube (26) et des première et seconde faces opposées, ladite ouverture (20) traversant lesdites première et seconde faces, ladite première face étant dotée d'une couche adhésive destinée à fixer ledit composant sparadrap (12) par rapport à ladite partie de surface cutanée dudit patient ou de ladite personne,
un composant support (30) disposé sur ladite seconde face dudit composant sparadrap (12) et faisant saillie par rapport à ladite seconde face, ledit composant support (30) étant disposé à proximité directe de ladite ouverture (20),
ledit composant support (30) ayant une partie de surface externe destinée à porter une longueur dudit tube (26) s'étendant de ladite ouverture (20) sur ladite seconde face dudit composant sparadrap (12) et étant configuré de telle sorte que ladite longueur dudit tube (26) est amenée en rotation d'une orientation sensiblement perpendiculaire à ladite partie de surface cutanée dudit patient ou de ladite personne à une orientation sensiblement parallèle à ladite partie de surface cutanée dudit patient ou de ladite personne,
le composant support (30) est constitué d'une coque mince en un matériau plastique affaissable élastiquement et déformable, et
ledit composant support (30) et ledit composant sparadrap (12) étant réunis,
le dispositif (10) étant **caractérisé en ce que**
la partie de surface externe du composant support (30) est configurée de telle façon que ladite longueur dudit tube (26) en relation de support avec ledit composant support (30) et portée latéralement par ladite partie de surface externe, est abaissée en contact avec la partie de surface cutanée du patient et mise en contact facial avec ledit composant sparadrap (12) et ladite partie de surface cutanée,
le composant support (30) définit une surface interne au sein de ladite coque, et
le composant support (30) comprend au moins un élément de renfort interne (33, 33', 33", 37, 37', 39) empêchant la compression ou la déformation du composant support (30) autour dudit au moins un élément de renfort (33, 33', 33", 37, 37', 39).

2. Dispositif selon la revendication 1, ledit au moins un élément de renfort (33, 33', 33", 37, 37', 39) étant un élément de support ou de renfort distinct positionné au sein de ladite coque ou reçu au sein dudit matériau plastique de ladite coque.

3. Dispositif selon la revendication 1, ledit élément de renfort (33, 33', 33", 37, 37', 39) étant une nervure de renfort intégrée positionnée au sein de ladite coque s'étendant le long et éventuellement parallèlement à au moins une partie de la partie de surface externe.

4. Dispositif selon la revendication 1, ledit élément de renfort (33, 33', 33", 37, 37', 39) étant positionné au sein de ladite coque et en dessous d'au moins une partie de ladite partie de surface externe.

5. Dispositif selon l'une quelconque des revendications 1 à 4, ledit matériau plastique étant capable de résister à l'exposition à un gaz stérilisant, tel que le gaz T.

6. Dispositif selon les revendications 4 ou 5, ledit composant support (30) ayant deux éléments de renfort ou nervures de renfort (33, 33', 33", 37, 37', 39) ou plus.

7. Dispositif selon l'une quelconque des revendications 1 à 6, ledit composant support (30) ayant un trou traversant (35) à travers ladite coque pour permettre au gaz de s'échapper par ledit trou lors de l'affaissement élastique ou de la compression de ladite coque.

8. Dispositif selon l'une quelconque des revendications 1 à 7, ledit élément de renfort interne (33, 37) s'étendant sur la longueur entière de ladite partie de surface externe.

9. Dispositif selon l'une quelconque des revendications 1 à 7, ladite partie de surface externe n'étant pas portée par ledit élément de renfort interne à proximité directe de ladite ouverture (20) dudit composant sparadrap (12).

10. Dispositif selon l'une quelconque des revendications 1 à 9, ledit composant support (30) comprenant en outre un ou plusieurs distanciers (41) positionnés au sein de ladite coque à une ou plusieurs positions et délimitant la hauteur de compression de ladite coque à ladite une ou plusieurs positions.

11. Dispositif selon l'une quelconque des revendications 1 à 10, ledit matériau plastique étant un copolymère éthylène/acrylate de butyle.

12. Dispositif selon l'une quelconque des revendications 1 à 11, ladite coque ayant une épaisseur d'approximativement 0,5 à 1,5 mm, tel que 0,6 à 1,0 mm, par exemple approximativement 0,6 à 0,8 mm ou une épaisseur de 0,6 à 0,7 mm, 0,7 à 0,8 mm, 0,8 à 0,9 mm, 0,9 à 1,0 mm, 1,0 à 1,1 mm, 1,1 à 1,2 mm, 1,2 à 1,3 mm, 1,3 à 1,4 mm ou 1,4 à 1,5 mm.

13. Dispositif selon l'une quelconque des revendications 1 à 12, ledit composant sparadrap (12) et ledit composant support (30) étant réunis au moyen d'un composant de connexion (38).

14. Dispositif selon la revendication 13, ledit composant de connexion (38) constituant une bride intégrée dudit composant support (30).

15. Dispositif selon l'une quelconque des revendications 13 ou 14, ledit composant de connexion (38) étant doté d'une ouverture, ladite ouverture étant en registre avec ladite ouverture (20) dudit composant sparadrap (12).

16. Dispositif selon l'une quelconque des revendications 1 à 15, ladite partie de surface externe dudit composant support (30) étant sensiblement rectiligne.

17. Dispositif selon l'une quelconque des revendications 1 à 16, ledit composant support (30) fournissant le support latéral dudit tube (26).

18. Dispositif selon l'une quelconque des revendications 1 à 17, comprenant en outre un moyen d'immobilisation (22) destiné à immobiliser ladite longueur dudit tube (26) par rapport audit composant support (30).

19. Dispositif selon la revendication 18, ledit moyen d'immobilisation (22) formant une partie intégrée dudit composant support (30).

20. Dispositif selon la revendication 18, ledit moyen d'immobilisation (22) étant connecté intégralement audit composant sparadrap (12).

21. Dispositif selon l'une quelconque des revendications 18 à 20, ledit moyen d'immobilisation (22) comprenant une bande adhésive destinée à fixer ledit tube (26).

22. Dispositif selon l'une quelconque des revendications 18 à 21, ledit moyen d'immobilisation (22) étant doté d'une zone transparente dans la zone de l'ouverture (20) dudit composant sparadrap (12).

23. Ensemble d'un tube (26) et d'un dispositif destiné à fixer ledit tube de drainage (26) par rapport à une partie de surface cutanée d'un patient ou d'une personne, ledit dispositif ayant l'une quelconque des caractéristiques du dispositif selon l'une quelconque des revendications 1 à 22.
